(19) Europäisches Patentamt
European Patent Office
Office européen des brevets

(11) **EP 0 904 532 B2**

(12) **NEUE EUROPÄISCHE PATENTSCHRIFT**

(45) Veröffentlichungstag und Bekanntmachung des Hinweises auf die Entscheidung über den Einspruch:
**21.11.2007 Patentblatt 2007/47**

(45) Hinweis auf die Patenterteilung:
**02.05.2002 Patentblatt 2002/18**

(21) Anmeldenummer: **97922799.8**

(22) Anmeldetag: **02.06.1997**

(51) Int Cl.:
*G01N 21/89* *(2006.01)*    *G01N 33/36* *(2006.01)*

(86) Internationale Anmeldenummer:
**PCT/CH1997/000222**

(87) Internationale Veröffentlichungsnummer:
**WO 1997/047959 (18.12.1997 Gazette 1997/54)**

(54) **VERFAHREN ZUR BEURTEILUNG DER AUSWIRKUNGEN VON GARNFEHLERN AUF TEXTILE FLÄCHENGEBILDE**

METHOD OF EVALUATING THE EFFECTS OF YARN DEFECTS ON TEXTILE SURFACE CONFIGURATION

PROCEDE POUR L'EVALUATION DES EFFETS DE DEFAUTS DU FIL SUR DES CONFIGURATIONS TEXTILES EN SURFACE

(84) Benannte Vertragsstaaten:
**BE CH DE FR GB IT LI**

(30) Priorität: **12.06.1996 CH 147296**

(43) Veröffentlichungstag der Anmeldung:
**31.03.1999 Patentblatt 1999/13**

(73) Patentinhaber: **Uster Technologies AG
8610 Uster (CH)**

(72) Erfinder: **FELLER, Peter
CH-8121 Benglen (CH)**

(74) Vertreter: **Ellenberger, Maurice
Uster Technologies AG
Wilstrasse 11
8610 Uster (CH)**

(56) Entgegenhaltungen:
EP-A- 0 578 975          WO-A-95/13519
DE-A- 2 744 241          DE-A- 4 131 664
DE-A- 4 341 685          US-A- 4 984 181
US-A- 5 319 578

• DATABASE WPI Section Ch, Week 8541 Derwent Publications Ltd., London, GB; Class F02, AN 85-253290 XP002024582 & JP 60 167 968 A (TEIJIN KK), 31.August 1985
• PATENT ABSTRACTS OF JAPAN vol. 96, no. 002 & JP 08 043318 A (KANEBO LTD), 16.Februar 1996,

**EP 0 904 532 B2**

**Beschreibung**

Die Erfindung betrifft ein Verfahren zur Beurteilung der Auswirkung von Garnfehlern auf textile Flächengebilde, durch Simulation eines Bildes des Flächengebildes ausgehend von einem vorgegebenen Garn.

Aus der EP-A-578975 ist ein solches Verfahren bekannt, bei dem in einem ersten Schritt ein Garn durch ein Messorgan auf Parameter untersucht wird, die mit dem Volumen und/oder mit der Oberfläche des Garns zusammenhängen. In einem zweiten Schritt werden die Parameter in Grau- oder Farbwerte umgerechnet und diese Werte werden Bildpunkten zugeordnet. Schliesslich werden die Bildpunkte auf einem Bildschirm und/oder einem Drucker wiedergegeben. Dadurch wird ein Bild erzeugt, welches eine Simulation eines aus dem untersuchten Garn hergestellten Gewebes oder Gewirkes darstellt.

Ein Nachteil des bekannten Verfahrens besteht nun darin, dass diese Simulation mit dem Bild eines wirklichen Gewebes oder Gewirkes nicht genau übereinstimmt, denn für die Simulation sind vereinfachende Annahmen getroffen worden, die sich in diesem Sinne auswirken. Eine solche Annahme besteht darin, in der Simulation parallel nebeneinanderliegende Garnabschnitte darzustellen und Bindungen zwischen Kettfäden und Schussfäden zu gewichten oder auch ganz zu vernachlässigen. Damit wird die Beurteilung eines simulierten Gewebes oder Gewirkes erschwert. Eine richtige Beurteilung setzt deshalb einige Übung voraus.

Die Aufgabe, die durch die Erfindung gelöst werden soll, besteht nun darin, die genannten Nachteile zu beheben und ein Verfahren zu schaffen, mit dem die Beurteilung simulierter textiler Flächengebilde mit grösserer Sicherheit erfolgen kann.

Die Aufgabe wird dadurch gelöst, dass ein Bild eines Gewebes oder Gewirkes, das durch eine bekannte Simulation entstanden ist, wobei Parameter eines wirklich existierenden und gemessenen Garns berücksichtigt wurden, mit dem Bild eines Referenzgewebes oder Referenzgewirkes verglichen wird, für das Parameter eines nach statistischen Gegebenheiten klassierten Referenzgarnes verwendet werden. Das Referenzgarn wird durch Parameter charakterisiert, die beispielsweise Durchschnittwerten entsprechen, wie sie aus publizierten Statistiken entnommen werden können. Dabei können die Parameter des Referenzgarnes durch Messung eines wirklich existierenden Referenzgarnes oder durch Berechnung aus gegebenen statistischen Werten gewonnen werden.

Mit anderen Worten soll ein Bild eines Referenzgewebes geschaffen werden, das dem Bild des Gewebes aus vorgegebenem Garn ähnlicher ist und mit dem das Bild des bekannten simulierten Gewebes verglichen werden kann. Das Bild des Referenzgewebes kann dabei durch Abbildung einer sogenannten Garnschautafel erreicht werden, die mit einem Referenzgarn aufgebaut ist. Oder, das Bild des Referenzgarnes kann durch Simulation eines Gewebes ausgehend von einem Referenzgarn erzeugt werden. Dabei geschiet die Simulation des Referenzgarnes durch Berechnung von Parametern des Garnes. Das beste Resultat erreicht man, wenn man zwei textile Flächengebilde auf gleiche Weise simuliert, wobei der einzige Unterschied in den Werten für die Parameter bestehen, wobei die einen Werte von einem vorgegebenen Garn und die anderen Werte von einem Referenzgarn stammen.

Die durch die Erfindung erreichten Vorteile sind insbesondere darin zu sehen, dass das Referenz-Gewebe oder Gewirke in genau gleicher Art und Weise wie das simulierte Gewebe oder Gewirke auf einem Bildschirm oder auf Papier dargestellt wird, so dass Abweichungen zwischen den Bildern direkt auf Abweichungen zwischen realen Gewirken und Geweben hindeuten und als Folge unterschiedlicher Werte für die gewählten Parameter angesehen werden. Für diese direkt vergleichbaren Bilder kann der Betrachter, der diese zu beurteilen hat nun auch eigene subjektive Kriterien anwenden mit denen er diese beurteilen will. Dabei muss er keine Verfälschung seiner Kriterien befürchten, wie sie dann zu berücksichtigen wären, wenn die Bilder nicht direkt vergleichbar sind.

Im folgenden wird die Erfindung anhand eines Beispiels und mit Bezug auf die beiliegenden Figuren näher erläutert. Es zeigen:

Figur 1 ein Bild einer Garnschautafel und einer simulierten Garnschautafel,

Figur 2 ein sogenanntes Stapeldiagramm,

Figur 3 mehrere Spektrogrammkurven für unterschiedliche Stapellängen,

Figur 4 eine Spektrogrammkurve für Garn aus zufällig verteilten Fasern verschiedener Länge,

Figur 5 eine Spektrogrammkurve die prozessbedingte langwellige Schwankungen berücksichtigt,

Figur 6 und 7 je eine Darstellung von Variationskurven,

Figur 8 eine schematische Darstellung einer inversen Fourier- Transformation,

Figur 9 eine Grafik im Zusammenhang mit seltenen Ereignissen im Garn,

Figur 10 eine Massen-Variationskurve für ein Garn und

Figur 11 ein Garnprüfgerät.

Das erfindungsgemässe Verfahren besteht einerseits aus der bekannten Simulation eines Bildes eines textilen Flächengebildes, das aus einem vorgegebenen Garn aufgebaut sein soll, dessen Parameter gemessen werden und andererseits aus der Simulation eines Bildes eines textilen Flächengebildes, das aus einem Referenzgarn aufgebaut ist. Hier gibt es die Möglichkeit, Parameter eines Referenzgarnes zu berechnen. Diese Möglichkeit soll nachfolgend mit bezug auf die Figuren genauer beschrieben werden. Mit den einzelnen Figuren sollen auch die notwendigen Verfahrensschritte erläutert werden.

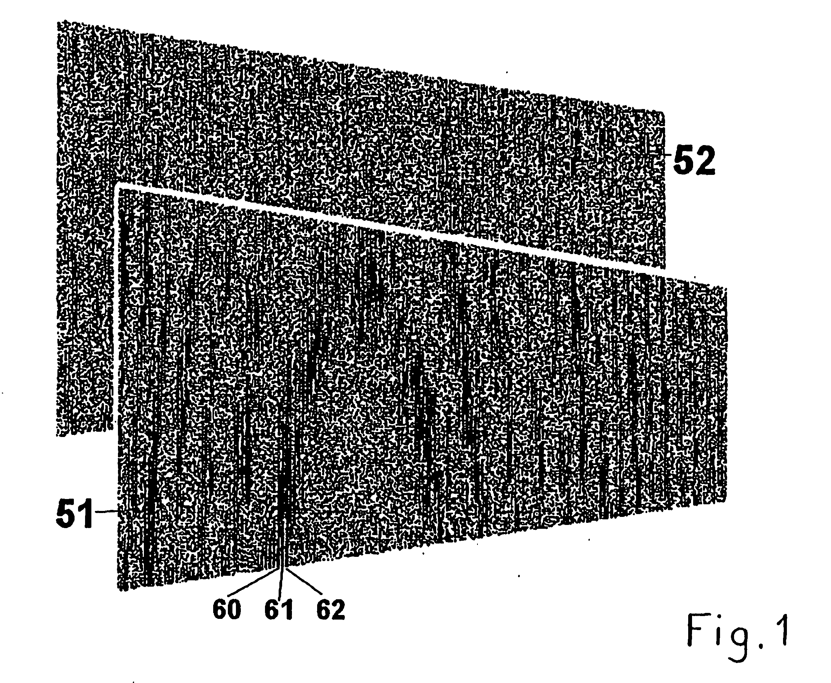

Fig. 1 zeigt ein Bild 52 eines Flächengebildes, das gemäss der vorliegenden Erfindung durch Simulation aus einem Referenzgarn aufgebaut ist. Dabei ist das Referenzgarn durch berechnete Parameter gegeben. Dieses Bild soll als Referenz für Bilder von Flächengebilden dienen, die aus anderen Garnen durch Simulation aufgebaut sind, wobei die Garne ausgehend von Messwerten an einem realen Garn simuliert sind. Ein solches Bild 51 ist zum Vergleich ebenfalls gezeigt. Ein Vergleich der beiden Bilder, wie er beispielsweise durch das menschliche Auge durchgeführt werden kann, erlaubt es Abweichungen in den Bildern 51, 52 zu erkennen und weiter auszuwerten. Beispielsweise ist im Bild 51 ein Moiré-Effekt erkennbar, dessen mögliche Ursachen an sich bekannt sind und beispielsweise auf periodische Fehler im Garn zurückzuführen ist.

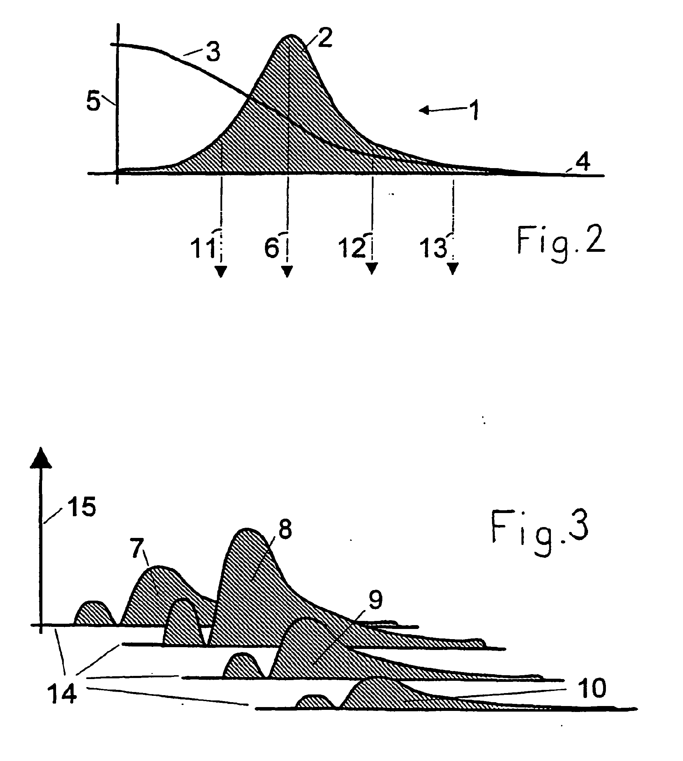

Fig. 2 zeigt ein sogenanntes Stapeldiagramm 1, in dem eine Kurve 2 für eine Häufigkeitsverteilung und eine Kurve 3 als Summenkurve der Häufigkeitsverteilung erkennbar ist. Das Stapeldiagramm ist Ausgangspunkt der Berechnung der Garnparameter. Die Kurven 2 und 3 sind über einer horizontalen Achse 4 aufgetragen, längs der Markierungen oder Werte für die Längen von textilen Fasern eingetragen sind. Längs einer vertikalen Achse 5 sind Markierungen oder Werte für den prozentualen Anteil von Fasern mit bestimmter Länge vorhanden. Aus der Kurve 2 ist somit leicht zu entnehmen, dass der grösste Anteil der Fasern eines Stapels aus dem die Werte stammen, die Länge entsprechend einem Wert an der Stelle 6 aufweisen. Aus der Kurve 3 kann man entnehmen, dass 100% der Fasern mindestens unendlich kurz sind, dass es aber keine unendlich langen Fasern gibt. Solche Stapeldiagramme sind entweder der Fachliteratur zu entnehmen oder können mit handelsüblichen Geräten für Rohstoffe gemessen werden. Solche Geräte werden durch die Firma Zellweger Uster unter der Bezeichnung AFIS oder AL100 vertrieben. Das Stapeldiagramm ist eine der Grundlagen der Berechnung der Garnparameter. Es liefert Werte die für einen Rohstoff, hier insbesondere ein Referenzrohstoff von dem man ausgeht, charakteristisch sind, d.h. es gibt an inwiefern für das Referenzgarn von einer idealen Verteilung der Faserlängen auszugehen ist. In Abhängigkeit seiner Zusammensetzung weist dann das Garn, das aus diesem Rohstoff hergestellt ist, mehr oder weniger ausgeprägte quasi- periodische Ungleichmässigkeiten auf, die in Spektrogrammen darstellbar sind.

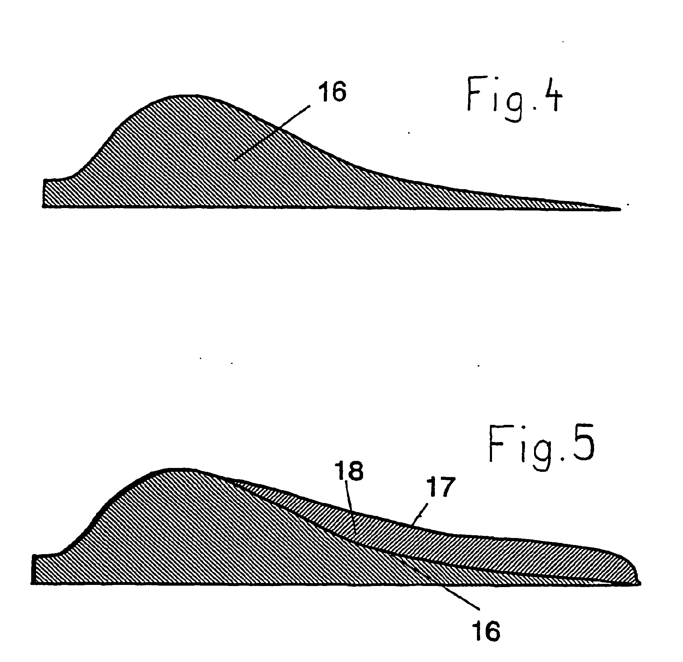

Fig. 3 zeigt in horizontal und vertikal verschobener Darstellung vier Spektrogrammkurven 7, 8, 9, 10 für je ein Garn, das aus Fasern konstanter Länge besteht, wobei die Länge der Fasern für diese Garne von der Spektrogrammkurve 7 zur Spektrogrammkurve 10 ansteigt. Längen von Fasern für diese Garne sind in Fig. 2 auf der Achse 4 beispielsweise an Stellen 6, 11, 12, und 13 abzulesen. Längs Achsen 14 sind Werte für Wellenlängen und längs einer Achse 15 sind Werte für Amplituden oder die Leistungsdichte (wie sie durch Gleichmässigkeitsprüfer erfasst wird) aufzutragen. Aus diesen Spektrogrammkurven 7 bis 10 erkennt man beispielsweise, dass die Amplituden der Unregelmässigkeiten im Garn mit langen Fasern relativ klein sind, wie dies die Spektrogrammkurve 10 zeigt, für kurze Fasern aber grösser sind, wie dies die Spektrogrammkurven 8 und 9 zeigen.

Für die Spektrogrammkurven wie sie durch bekannte Gleichmässigkeitsprüfer für Garn ausgegeben werden, gilt die Formel

$$(A) \qquad p(f) = \frac{1}{n} \sin^2\left(\frac{\Pi f L}{v}\right)$$

wobei
p die Leistungsdichte, wie sie in einem bekannten Gleichmässigkeitsprüfer für Garn gemessen wird,
n die Anzahl der Fasern im Querschnitt des Garns,
L die Länge der Fasern
f die Messfrequenz und
v die Messgeschwindigkeit des Garns bedeuten.
Bei einigen Gleichmässigkeitsprüfern wird die Leistung mit Bandfiltern gemessen, die konstante relative Bandbreite aufweisen. Beispielsweise sind pro Oktave 5 Bandfilter angeordnet, deren Bandgrenzen sich berühren. So ergibt sich statt der hier gezeigten kontinuierlichen Kurve eine treppenförmige Spektrogrammkurve.

Für ein Spektrogramm in kontinuierlicher Form gilt Formel

$$(B) \qquad a\,(\log \lambda) = c\,\frac{\sin(\frac{\Pi L}{\lambda})}{\sqrt{\lambda/L}}$$

wobei

c eine Proportionalitätskonstante ist.

Da letztlich für den Vergleich der simulierten Flächengebilde einerseits an bekannten Garnprüfern gemessene Parameter verwendet werden, ist es zweckmässig auch für die Simulation des Referenzgarnes und des Referenzbildes andererseits möglichst mit Werten zu arbeiten wie sie in den Garnprüfem ermittelt werden.

So liefern bekannte Garnprüfer Darstellungen von Spektrogrammen bei denen statt der Leistungsdichte die Wurzel der Leistung oder statt der Frequenz die Wellenlänge in logarithmischem Massstab angegeben ist. Dies ist bei der gezeigten Darstellung der Formel (B) berücksichtigt.

Aus den Spektrogrammkurven 7 bis 10 für Garne, die aus Fasern gleicher Länge aufgebaut sind, soll eine Spektrogrammkurve für ein Garn abgeleitet werden, das aus Fasern mit unterschiedlichen Längen besteht wie dies ja bei realen Garnen üblich ist.

Fig. 4 zeigt deshalb eine Spektrogrammkurve 16, die durch Überlagerung mehrerer Spektrogrammkurven wie z.B. die Spektrogrammkurven 7, 8, 9, 10 entstanden ist. Aus dem Stapeldiagramm (Fig. 1) können für Faserlängenwerte, die beispielsweise in konstanten Abständen längs der Achse 14 vorgegeben werden, Werte für die Häufigkeit aus den Kurven 2 oder 3 entnommen werden. Mit diesen Werten können die Spektrogramme für die betreffenden Faserlängen gewichtet werden. Die Spektrogrammkurve 16 wird gemäss der nachstehenden Formel (C) für die gewichtete Spektrogrammkurve berechnet:

$$(C) \qquad a\,(\log \lambda) = c\,\sqrt{\sum_{i=o}^{n} h_i \frac{\sin^2(\pi\,\lambda\,/\,(L_0\,k^i))}{\lambda\,/\,(L_0\,k^i)}}$$

k der Logarithmus des Längenverhältnisses benachbarter Klassen $L_i/L_{i+1}$ und

h die Häufigkeit der Faserzahl in Abhängigkeit von der Faserlänge, wie sie aus der Fig. 2 zu entnehmen ist, bedeutet.

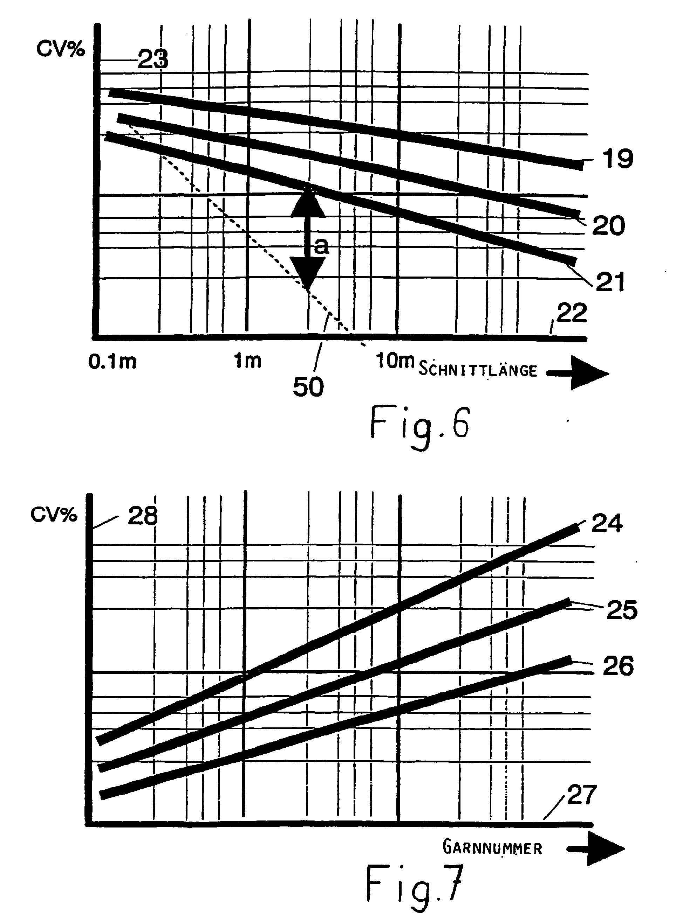

Fig. 5 zeigt eine Spektrogrammkurve 17, die aus der Spektrogrammkurve 16 abgeleitet ist. In der Spektrogrammkurve 17 sind Fehler berücksichtigt, die ein Garn als Folge von Produktionsbedingungen aufweist, die nicht ideal sind. Solche, beispielsweise durch Produktionsmaschinen bewirkte oder nicht behobene Fehler sind meistens langwellig, weshalb die Spektrogrammkurve 17 insbesondere in einem Bereich 18 von der Spektrogrammkurve 16 abweicht. Die Abweichung im Bereich 18 kann durch Werte aus bekannten Längenvariationskurven CV(L) und Massenvariationskurven CVm ermittelt werden, wie nachfolgend noch dargelegt wird.

Fig. 6 zeigt drei Grenzen 19, 20 und 21, die Felder begrenzen, in denen Längenvariationskurven CV(L) für Garne unterschiedlicher Qualität liegen können. Diese sind über einer horizontalen Achse 22 mit Werten für Schnittlängen der Garne und neben einer vertikalen Achse 23, mit Werten für prozentuale Abweichungen von einem Mittelwert, aufgetragen. Die Grenze 19 betrifft Garne der schlechtesten, Grenze 21 Garne der besten Qualität. Daraus erkennt man, dass bei Garnen guter Qualität die Abweichungen vom Mittelwert mit zunehmender Schnittlänge stärker abnehmen, als für Garne schlechter Qualität. Zusätzlich ist eine Längenvariationskurve 50 eines idealen Garnes eingezeichnet. Da gemäss den Figuren 3 und 4 bekannt ist, dass langwellige Fehler im Garn kleinere Amplitude haben als kurzwellige Fehler, gibt die Fig. 6 einen Hinweis darauf, dass bei Garnen schlechter Qualität wegen schlechter Produktionsmaschinen auch langwellige Fehler noch Amplituden haben, die nicht vernachlässigt werden können. Deshalb müssen die Amplitudenwerte aus der Spektrogrammkurve 16 mit einem Faktor korrigiert bzw. multipliziert werden, der die real vorhandenen langwelligen Fehler im nicht idealen Garn berücksichtigt. Diese Korrektur soll im abfallenden Ast der Spektrogrammkurve 16 insbesondere für Schnittlängen über ca. 0.5 m im Bereiche 18 erfolgen. Der Faktor wird für verschiedene Schnittlängen gebildet und ergibt sich aus dem Abstand a zwischen der Längenvariationskurve 50 und der gewählten Grenze 21, 20, 19. Da längs beiden Achsen 22, 23 die Werte logarithmisch aufgetragen sind, kann dieser Abstand a durch Entlogarithmisierung direkt in einen Faktor umgewandelt werden. Durch diese Korrektur entsteht aus der Spektrogrammkurve 16 die Spektrogrammkurve 17.

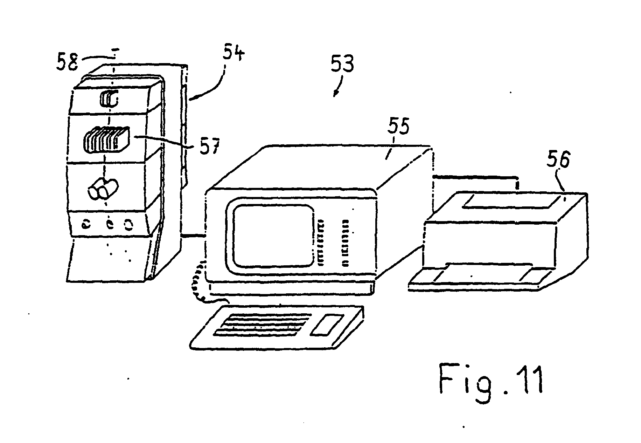

Aus dem vorliegenden Spektrogramm 17 soll nun durch Berechnung ein Garnsignal hergestellt werden, wie es auch ein Garnprüfer ausgeben könnte. Dies wird in Fig. 8 dargestellt. Dazu wird eine inverse Fourier-Transformation eingesetzt, die aus Signalen im Spektralbereich zu einem Ausgangssignal führen soll, das Querschnitts- oder Massenabweichungen längs des Garns darstellt. Dazu wird die Spektrogrammkurve 17 logarithmisch in Klassen aufgeteilt, die hier durch Rechtecke 29 bis 35 dargestellt sind. In logarithmischern Massstab weisen diese Klassen unter sich gleiche Längen auf. Jede Klasse 29 bis 35 stellt somit auch einen Wellenlängenbereich dar, der auch weiter in mehrere Kanäle unterteilt werden kann, so z.B. in 5 bis 10 Kanäle pro Oktave. Jeder Klasse 29 bis 35 ist ein Sinusgenerator 36 bis 42 zugeordnet, dessen Frequenz umgekehrt proportional zur Wellenlänge der Klasse und dessen Amplitude proportional zur Höhe der Klasse, bzw. zur Häufigkeit (entsprechend der Höhe des Rechteckes) der durch die Klasse dargestellten Abweichungen ist. Jeder Generator 36 bis 42 gibt somit ein sinusförmiges Signal aus, die durch Überlagerung zusammengemischt werden, so dass ein einziges Ausgangssignal entsteht, das Massenabweichungen von einem Mittelwert über die Zeit darstellt, wie es beispielsweise in der Fig. 10 gezeigt ist. Für dieses Ausgangssignal kann ein Variationskoeffizient in an sich bekannter Weise ermittelt werden.

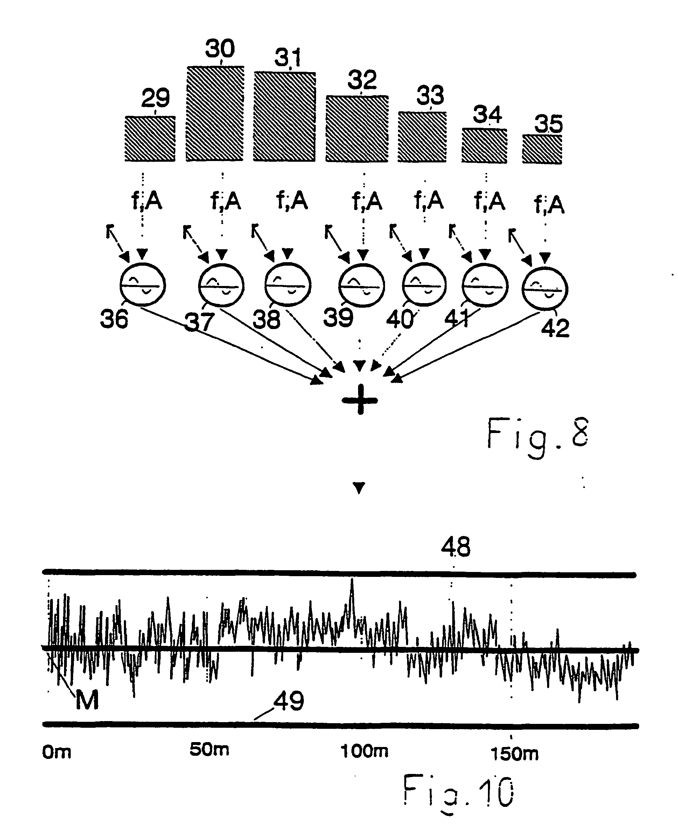

Solche Variationskoeffizienten sind auch in der Fig. 7 für bekannte Garne aufgezeichnet Fig. 7 zeigt drei Grenzen 24, 25 und 26 innerhalb denen Werte der Massenvariation für Garne unterschiedlicher Qualität liegen können. Diese sind über einer horizontalen Achse 27 mit Werten für die sogenannte Garnnummer oder Feinheit (welche zur Dicke invers proportional ist) und neben einer Achse 28, mit Werten für Variationskoeffizienten CV in Prozenten von einem Mittelwert, aufgetragen. Die Grenze 24 betrifft Garne der schlechtesten, Grenze 26 Garne der besten Qualität. Daraus erkennt man, dass bei Garnen guter Qualität die Abweichungen vom Mittelwert mit zunehmender Garnnummer schwächer zunehmen, als für Garne schlechter Qualität. Nun hat man ja eine Vorstellung über die Qualität des Garns das man simulieren möchte. Aus dieser Vorstellung kann für das betreffende Garn aus der Fig. 7 für eine bestimmte Garnnummer der Variationskoeffizient aus einer der Grenzen 24, 25, 26 entnommen werden. Vergleicht man diesen mit dem Variationskoeffizienten für das Ausgangssignal gernäss Fig. 8, so stellt man wahrscheinlich einen Unterschied fest. Daraus ermittelt man einen Faktor, mit dem das gesamte Ausgangssignal multipliziert wird, mit dem also jede Auslenkung des Signales zu einer Zeit vergrössert oder verkleinert wird. So entsteht ein simuliertes Signal, das weitgehend an reale Umstände angepasst ist.

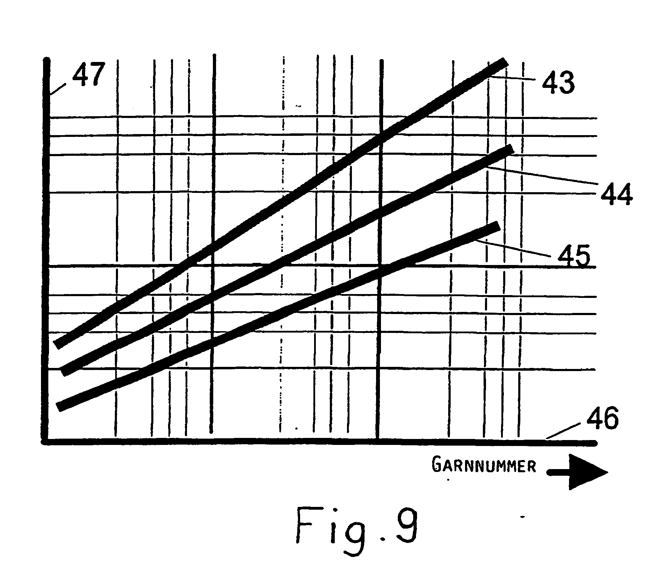

Die Anpassung der Simulation des Garnes an reale Umstände kann aber noch weiter getrieben werden. Dazu kann zusätzlich die Frequenz jedes Sinusgenerators (Fig. 8) vor dem Mischen noch mit einem Zufallssignal in der Frequenz moduliert werden, so dass ein breitbandigeres Signal entsteht. Die Bandbreite des Zufallssignals entspricht vorzugsweise den Kanalabständen. Allerdings weisen Garne meist noch sogenannte seltene Ereignisse wie Nissen, Dickstellen, Dünnstellen oder Fremdkörper auf, die bis dahin nicht berücksichtigt wurden. Solche Ereignisse können mit einem Zufallsgenerator simuliert und dem Signal beigemischt werden. Die Häufigkeit und Grösse solcher Ereignisse ist beispielsweise der Publikation "USTER STATISTICS" für die betreffenden Ereignisse zu entnehmen. Dem Zufallsgenerator können Häufigkeitswerte eingegeben werden, wie sie beispielsweise aus der Fig. 9 entnommen werden können.

Fig. 9 zeigt ein Beispiel einer Grafik wie sie in den oben erwähnten USTER STATISTICS neben Grafiken gemäss den Figuren 6 und 7 zu finden ist. Diese zeigt drei Grenzen 43, 44 und 45 für Werte, die eine Anzahl seltener Ereignisse pro Garnlänge angeben. Diese Werte und Grenzen sind über einer horizontalen Achse 46 mit Werten für die Garnnummer und neben einer Achse 47, mit Werten für die Anzahl Ereignisse pro 1000 Meter Garn aufgetragen. Die Grenze 43 betrifft Garne der schlechtesten, Grenze 45 Garne der besten Qualität. Daraus erkennt man, dass bei Garnen guter Qualität die Anzahl Ereignisse mit zunehmender Garnnummer schwächer zunimmt, als bei Garnen schlechter Qualität. Die Beimischung mit dem Zufallsgenerator kann mit einer zufällig erzeugten Amplitude und Länge, die die Abweichung des Ereignisses vom Mittelwert quantifiziert, erfolgen. Der Zufallsgenerator gibt dann Impulse aus, die dem Ausgangssignal gemäss Fig. 8 überlagert werden und die einem Erfahrungswert typischer Imperfektionen entsprechen. Fig. 9 stell nur ein Beispiel von vielen Statistiken dar, die die Häufigkeit von speziellen Imperfektionen wie Dickstellen, Dünnstellen, Nissen, Schalenteile, Frerndstoffe usw auch getrennt angeben.

Fig. 10 zeigt beispielsweise eine an sich für Garn bekannte Variationskurve 48 für Massenvariationen, die von einem Mittelwert M abweichend dargestellt sind. Das mit den Figuren 2 bis 8 dargestellte Verfahren liefert nun eine solche Variationskurve 48, die über einer Achse 49 aufgezeichnet ist, auf der Werte für die Garnlänge aufgetragen sind. Zu jeder einzelnen Variation, die durch eine vertikale Abweichung dargestellt ist, deren Höhe dem Ausmass der Abweichung vom Mittelwert M entspricht, ist auch die Lage im Garn oder längs des Garns bekannt. Die Variationskurve 48 unterscheidet sich in ihrer Art von einer Variationskurve, die durch Messung eines Garns in einem Garnprüfer ermittelt wurde nicht So können die einzelnen Variationen oder Signalpunkte oder die Werte die sie darstellen, direkt in Bildpunkte gewandelt und aneinandergereiht werden, so dass eine Simulation eines Garns entsteht. Die Abweichungen der Signalpunkte geben dabei ein Mass für die Intensität einer Farbe oder eines Grauwertes an. Werden nun mehrere Reihen solcher Bildpunkte aneinandergereiht, so entsteht beispielsweise das Bild 51 (Fig. 1) aus dem klar einzelne Reihen 60, 61, 62 erkennbar sind, die aus Bildpunkten zusammengesetzt sind, die hier nur in zwei Intensitäten oder Abstufungen, d.h. schwarz oder weiss erscheinen. Auch das Bild 52 entsteht in gleicher Weise, lediglich mit dem Unterschied, dass die Parameter oder das Messsignal, die in Bildpunkte umgewandelt wurden, von einem Garn stammen das als Referenz dient und durch Messung des Garns in einem Garnprüfer oder durch Simulation ermittelt wurden. In entsprechender Weise sind auch Simulationen für Gewebe mit verschiedenen Bindungen möglich, beispielsweise für Gewirke. Dann sind die Bildpunkte eines Garnes im Bild entsprechend dem Verlauf des Garnes im betreffenden Flächengebilde angeordnet. So wie in Geweben sich die Kettund Schussfäden kreuzen und damit überdecken, so bilden die Fäden oder Garne im Gewirke Maschen. In beiden Fällen kann man die Überdeckung in der Simulation, durch verstärkte Intensität an dieser Stelle, berücksichtigen oder nicht.

Fig. 11 zeigt ein an sich bekanntes Garnprüfgerät 53, das aus dem eigentlichen Prüfgerät 54, einer Auswerte- und Bedienungseinheit 55 und einem Drucker 56 besteht. Das Prüfgerät 54 ist mit einem oder mehreren Messmodulen 57 versehen, welche Messorgane für die zu untersuchenden Parameter aufweisen. Ein Garn 57, dessen Parameter, wie z.B. die Masse, die Haarigkeit oder die Struktur fortlaufend gemessen werden sollen, wird in bekannter Weise durch die Messorgane transportiert.

Das erfindungsgemässe Verfahren kann somit in eine Anzahl Schritte zusammengefasst wie folgt dargestellt werden:

a) Erzeugen von Bildpunkten eines Referenzgarnes durch Berechnung mindestens eines relevanten Parameters aus statistischem Material wie Durchschnittswerten für Garne und anschliessendes Umwandeln der Werte des Parameters in Werte für die Intensität von Bildpunkten, die aneinandergereiht eine Simulation für das Referenzgarn ergeben. Dabei gehört zu jedem Bildpunkt auch eine Information über seine Lage längs des Referenzgarns.

b) Erzeugen von Bildpunkten eines vorgegebenen Garnes durch Messung mindestens eines relevanten Parameters am vorgegebenen Garn und anschliessendes Umwandeln der Werte des Parameters in Werte für die Intensität von Bildpunkten, die aneinandergereiht eine Simulation für das vorgegebene Garn ergeben. Dabei gehört zu jedem Bildpunkt auch eine Information über seine Lage längs des vorgegebenen Garns.

c) Erzeugen eines ersten Bildes eines textilen Flächengebildes durch Anordnen der Bildpunkte eines Garnes entsprechend dem Garnverlauf im Flächengebilde, z.B. durch Nebeneinanderlegen der Reihen der Bildpunkte eines vorgegebenen Garns, dessen Parameter gemessen sind.

d) Erzeugen eines zweiten Bildes eines textilen Flächengebildes durch Anordnen der Bildpunkte eines Garns entsprechend dem Garnverlauf im Flächengebilde, z.B. durch Nebeneinanderlegen der Reihen der Bildpunkte des Referenzgarnes.

e) Vergleich des ersten und des zweiten Bildes indem diese nebeneinander oder übereinander abgebildet werden. Hier besteht auch die Möglichkeit, den Inhalt des ersten Bildes durch den Inhalt des zweiten Bildes auszutauschen und umgekehrt, oder kontinuierlich die Bilder zu überblenden.

Es besteht auch die Möglichkeit, statt von statistischen Werten auszugehen, wie sie in den Stapeldiagrammen und Spektrogrammen enthalten sind, von einer Simulation eines Garnsignales auszugehen. Dabei könnte jede Faser durch einen Rechteckimpuls mit der Länge der Faser ersetzt und durch zufällige Verteilung dieser Rechteckimpulse ein Garnsignal erzeugt werden. Zusätzlich zu der Simulation von Flächengebilden wie Geweben, Gewirken usw. kann so auch eine Simulation von sogenannten Garnschautafeln erfolgen. So kann je ein Bild einer Garnschautafel mit einem Referenzgarn und einer Garnschautafel mit einem gegebenen Garn erzeugt werden. Auch hier kann sowohl das Referenzgarn wie auch das gegebene Garn durch gemessene Parameter oder berechnete Parameter simuliert werden.

Man kann davon ausgehen, dass die gesamte Berechnung des Garnsignals für das Referenzgarn in einem Computer durchgeführt werden kann, der durch einen Fachmann zu diesem Zweck und zur Durchführung von Operationen wie sie anhand der Figuren 2 bis 10 dargestellt wurden programmiert werden kann.

Das erfindungsgemässe Verfahren kann in einer Vorrichtung, wie sie die Fig. 11 zeigt, durchgeführt werden, wenn die Auswertungs- und Bedienungseinheit 55 ein entsprechendes Programm aufweist. Sind darin beispielsweise die Parameter für ein- oder mehrere Referenzgarne gespeichert, so kann jederzeit ein Bild eines Referenzgewebes- oder Gewirkes auf dem Bildschirm erzeugt werden. Daneben können Parameter für ein reales Garn 58 im Messmodul 57 geprüft werden und ebenfalls in der Auswertungs- und Bedienungseinheit 55 zu einem Bild für ein simuliertes Gewebe oder Gewirke führen. Mit hilfe von bekannten Programmen zur Bildverarbeitung und Bilddarstellung können die beiden Bilder auf dem Bildschirm oder durch den Drucker 56 so ausgegeben werden, dass ein Vergleich gut möglich ist.

**Patentansprüche**

1. Verfahren zur Beurteilung der Auswirkung von Garnfehlern auf textile Flächengebilde, durch Simulation eines Bildes des Flächengebildes ausgehend von einem vorgegebenen Garn, wobei ein erstes Bild (51) des Flächengebildes durch eine Simulation ausgehend von Parametern des vorgegebenen Garnes erzeugt wird, **dadurch gekennzeichnet dass** ein zweites Bild (52) des Flächengebildes durch eine Simulation ausgehend von Parametern eines Referenzgarnes erzeugt wird, dass die Parameter des Referenzgarnes durch Berechnung aus statistisch ermittelten Werten ermittelt werden und dass ein Vergleich des ersten Bildes mit dem zweiten Bild durchgeführt wird.

2. Verfahren nach Anspruch 1, **dadurch gekennzeichnet, dass** die Parameter des vorgegebenen Garnes (58) durch Messung des Garnes in einem Garnprüfgerät (53) ermittelt werden, wobei für einen Parameter mehrere Werte ermittelt und zu jedem Wert eine Angabe über den Ort längs des Garns gemacht wird, auf den ein Wert zutrifft.

3. Verfahren nach Anspruch 1, **dadurch gekennzeichnet, dass** durch Berechnung für einen Parameter eine Variationskurve (48) ermittelt wird.

4. Verfahren nach Anspruch 3, **dadurch gekennzeichnet, dass** als Parameter die Masse gewählt wird und dass eine Massenvariationskurve (48) ermittelt wird.

5. Verfahren nach Anspruch 2 und 3, **dadurch gekennzeichnet, dass** Werten aus den Variationskurven Grau-/oder Farbwerte zugeordnet werden.

6. Verfahren nach Anspruch 5, **dadurch gekennzeichnet, dass** Grau-/oder Farbwerte in Bildpunkte umgesetzt und in Reihen (60, 61, 62) aneinandergereiht werden um ein Garn zu simulieren und dass die Reihen entsprechend dem Garnverlauf im Flächengebilde angeordnet werden um ein Flächengebilde zu simulieren.

7. Verfahren nach Anspruch 1, **dadurch gekennzeichnet, dass** aus Spektrogrammkurven (7, 8, 9, 10) und aus Stapeldiagrammen (1) Variationskurven (48) für ein simuliertes Garn gewonnen werden.

8. Verfahren nach Anspruch 7, **dadurch gekennzeichnet, dass** seltene Ereignisse in den Variationskurven (48) berücksichtigt werden.

**Claims**

1. A method for assessing the effect of yarn defects on textile fabrics, by simulating an image of the fabric based on a given yarn, wherein a first image (51) of the fabric is generated by simulation based on parameters of the given yarn, **characterized in that** a second image (52) of the fabric is generated by simulation based on parameters of a reference yarn, **in that** the parameters of the reference yarn are determined by calculation from statistically determined values, and **in that** the first image is compared with the second image.

2. A method according to claim 1, **characterized in that** the parameters of the given yarn (58) are determined by measuring the yarn in a yarn testing apparatus (53), wherein several values are determined for one parameter and for each value an indication is given of the place along the yarn to which a value relates.

3. A method according to claim 1, **characterized in that** a variation curve (48) for a parameter is determined by calculation.

4. A method according to claim 3, **characterized in that** the mass is selected as a parameter and **in that** a mass variation curve (48) is determined.

5. A method according to claim 2 and 3, **characterized in that** grey or colour values are assigned to values from the variation curves.

6. A method according to claim 5, **characterized in that** grey or colour values are converted into pixels and strung together in rows (60, 61, 62) to simulate a yarn and **in that** the rows are arranged according to the yarn flow in the fabric to simulate a fabric.

7. A method according to claim 1, **characterized in that** variation curves (48) for a simulated yarn are obtained from spectrogram curves (7, 8, 9, 10) and from staple diagrams (1).

8. A method according to claim 7, **characterized in that** rare occurrences are taken into account in the variation curves (48).

**Revendications**

1. Procédé pour l'évaluation des effets de défauts du fil sur des configurations textiles en surface, par la simulation d'une image de la configuration surfacique en partant d'un fil prédéterminé, où une première image (51) de la configuration surfacique est produite par une simulation en partant de paramètres du fil prédéterminé, **caractérisé en ce qu'**une seconde image (52) de la configuration surfacique est produite par une simulation en partant de paramètres d'un fil de référence, que les paramètres du fil de référence sont déterminées par un calcul à partir de valeurs obtenues d'une manière statistique et qu'une comparaison de la première image avec la seconde image est effectuée.

2. Procédé selon la revendication 1, **caractérisé en ce que** les paramètres du fil prédéterminé (58) sont déterminés par la mesure du fil dans un appareil de contrôle de fil (53) où pour un paramètre, plusieurs valeurs sont déterminées et, pour chaque valeur, une indication est faite sur le lieu le long du fil auquel s'applique une valeur.

3. Procédé selon la revendication 1, **caractérisé en ce que** par le calcul pour un paramètre, une courbe de variation (48) est obtenue.

4. Procédé selon la revendication 3, **caractérisé en ce que**, comme paramètre, la masse este sélectionnée et qu'une courbe de variation de masse (48) est obtenue.

5. Procédé selon la revendication 2 et 3, **caractérisé en ce que** des valeurs de gris ou de couleur sont associées aux valeurs des courbes de variation.

6. Procédé selon la revendication 5, **caractérisé en ce que** des valeurs de gris ou de couleur sont transformées en points d'image et sont juxtaposées en rangées (60, 61, 62) pour simuler un fil et **en ce que** les rangées sont disposées conformément au périple du fil dans la configuration surfacique pour simuler une configuration surfacique.

7. Procédé selon la revendication 1, **caractérisé en ce que** sont obtenues à partir de courbes de spectrogrammes (7, 8, 9, 10) et de diagrammes d'empilage (1) des courbes de variation (48) pour un fil simulé.

8. Procédé selon la revendication 7, **caractérisé en ce que** des événements rares dans les courbes de variation (48) sont pris en considération.

Fig. 1

Fig. 2

Fig. 3

Fig.4

16

Fig.5

18    17

16

Fig.6

Fig.7

Fig. 8

Fig. 10

GARNNUMMER ➡

Fig.9

Fig.11

**EP 0 904 532 B2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 578975 A